# EUROPÄISCHE PATENTSCHRIFT

(11) **EP 1 062 349 B1**
(45) Veröffentlichungstag und Bekanntmachung des Hinweises auf die Patenterteilung: **11.06.2003**
(21) Anmeldenummer: 99915534.4
(22) Anmeldetag: 23.02.1999
(51) Int. Cl.: C12N 15/55, C12N 9/16, C12N 15/70, C12N 15/81, C12N 1/21, C12N 1/19, C12N 11/14, C12N 11/16, C12P 21/02, C12Q 1/42, A61K 38/46

(54) **DIISOPROPYLFLUOROPHOSPHATASE SOWIE DEREN VERWENDUNG UND HERSTELLUNG**
DIISOPROPYL FLUOROPHOSPHATASE AND THE UTILIZATION AND PRODUCTION THEREOF
DI-ISOPROPYLFLUOROPHOSPHATASE, SON UTILISATION ET SA PREPARATION

(30) Priorität: 27.02.1998 DE 19808192
(43) Veröffentlichungstag der Anmeldung: 27.12.2000
(73) Patentinhaber: Roche Diagnostics GmbH, 68305 Mannheim (DE)
(72) Erfinder: RÜTERJANS, Heinz, D-61352 Bad Homburg (DE); DIERL, Stefan, D-65835 Lieferbach (DE)
(74) Vertreter: Leifert, Elmar, Dr.
(86) Internationale Anmeldenummer: EP9901159
(87) Internationale Veröffentlichungsnummer: WO99043791

(56) Entgegenhaltungen:
- HOSKIN, F.C.G. AND ROUSH, A.H.: "Hydrolysis of nerve gas by squid-type diisopropyl phosphorofluoridate hydrolyzing enzyme on agarose resin" SCIENCE, Bd. 215, Nr. 4537, 5. März 1982 (1982-03-05), Seiten 1255-1257, XP002126689 in der Anmeldung erwähnt
- MCGUINN, W.D. ET AL.: "The encapsulation of squid diisopropylphosphorofluoridate- hydrolyzing enzyme within mouse erythrocytes" FUNDAMENTAL AND APPLIED TOXICOLOGY, Bd. 21, 1993, Seiten 38-43, XP002126690 in der Anmeldung erwähnt
- STEINMAN, K.E.: "Characterization of multiple forms of squid organophosphorus acid anhydrase" DISSERTATION ABSTRACTS INTERNATIONAL, Bd. 51, Nr. 5, November 1990 (1990-11), Seite 2161-B XP002126691
- HOSKIN, F.C.G.: "Squid nerve type DFPase: a consideration of molecular structures" PROCEEDINGS OF THE JERUSALEM SYMPOSIUMON QUANTUM CHEMISTRY AND BIOCHEMISTRY, Bd. 7, 1974, Seiten 209-211, XP002126692
- WANG, F. ET AL.: "Purification and properties of a diisopropyl- fluorophosphatase from squid Todarodes Pacificus Steenstrup" JOURNAL OF BIOCHEMICAL TOXICOLOGY, Bd. 8, Nr. 3, 1993, Seiten 161-166, XP002126693 in der Anmeldung erwähnt

## Beschreibung

Die vorliegende Erfindung betrifft eine Diisopropylfluorophosphatase (Enzym-Klassifikation EC 3.1.8.2., im folgenden DFPase genannt) aus *Loligo vulgaris*, die das Enzym kodierende Basensequenz, sowie die Verwendung des Enzyms und ein Verfahren zu dessen Herstellung in transformierten Zellen.

Der Begriff DFPase besitzt eine weite Verbreitung bei der Beschreibung einer Klasse von Enzymen, welche in der Lage sind, Diisopropylfluorophosphat (DFP) und andere phosphororganische Verbindungen mit ähnlicher Struktur zu hydrolysieren. Die Gruppe dieser phosphororganischen Verbindungen läßt sich auf Verbindungen der folgenden Grundstruktur zurückführen:

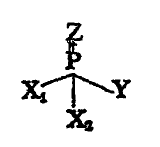

Hierin steht Z für Sauerstoff oder Schwefel, Y steht für eine Gruppe, die als H-Y-Verbindung acide Eigenschaften haben kann. Unter anderem sind dies Säureanhydridgruppen (deprotonierte Säuregruppen) (insbesondere eine F- oder CN-Gruppe) oder Estexgruppierungen, wie eine Thioester-, eine Enolester oder eine p-Nitrophenylestergruppe. Bei den Gruppierungen X₁ und X₂ kann es sich um gerad- oder verzweigtkettige oder cyclische, 1 bis 15 Kohlenstoffatome enthaltende, Alkoxy-, Alkyl-, Aryl-, Alkylamino- oder Dialkylaminogruppen handeln. Eine Vielzahl derartiger Verbindungen sind oder waren als Insektizide im Gebrauch. Andere Verbindungen fallen unter den Begriff der sog. Nervengase. Zu den Nervengasen dieser Verbindungsklasse zählen u. a. DFP, Tabun, Soman, Sarin, Ethylsarin und Cyclosarin.

Der Abbau der weltweiten Vorräte dieser hochgiftigen Verbindungen stellt ein zunehmendes Problem dar. Darüber hinaus müssen umweltschonende Methoden gefunden werden, bereits verseuchte Bereiche der Umwelt zu dekontaminieren.

Das bisher wichtigste Verfahren zur Vernichtung großer Mengen dieser Substanzen stellt die Verbrennung bei hohen Temperaturen dar.

Darüber hinaus beschäftigt sich z. B. US-A-4,666,696 mit der Zerstörung von Nervengasen und anderen Cholinesterase-Inhibitoren durch Reduktion mit geschmolzenem Aluminium.

Weitere Ansätze zur Lösung dieses Problems bestehen z. B. im Einsatz physikalischer Methoden. So beschreibt z. B. US-A-5,550,311 die thermische Zersetzung toxischer Verbindungen (Nervengase) im Wasserstrahl. Ein anderer Ansatz ist in der US-A-5,648,591 beschrieben, in welcher Chemiewaffen, wie z. B. Sarin, durch Aktivierung in mechanischen Mühlen abgebaut werden. All diese Methoden weisen bereits starke Nachteile bezüglich ihrer Wirtschaftlichkeit und ihres begrenzten Anwendungsspektrums auf. So ist z. B. in all diesen Verfahren die Notwendigkeit gegeben, die entsprechenden toxischen Substanzen bestimmten Apparaten zuzuführen, wodurch z. B. ein Feldeinsatz nur schwer, wenn nicht gar unmöglich ist.

Die biologische Entsorgung der C-Waffenbestände und die Dekontamination durch Mikroorganismen oder Enzyme, die man in großen Mengen herstellen kann, könnte eine Vereinfachung oder einen effektiveren Lösungsansatz dieser Problematik bieten.

Den oben beschriebenen Dekontaminationsmethoden steht daher die vorliegende Erfindung gegenüber, deren Hintergrund bereits auf Untersuchungen aus dem Jahr 1953 durch Aldridge aufbaut. Aldridge beobachtete, daß verschiede Gewebe unterschiedlicher Organismen Enzyme enthalten, die Paraoxon, einen Cholinesterase-Inhibitor, hydrolysieren. Spätere Untersuchungen aus dem Jahr 1966 durch Hoskin und Mitarbeiter zeigten, daß im Tintenfisch *Loligo pealii* ein DFP-spaltendendes Enzym enthalten ist. Bis heute folgten zahlreiche Untersuchungen auf diesem Gebiet, die Sequenzierung oder gar Gewinnung eines rekombinanten Proteins gelang bisher jedoch nicht. Das von Hoskin untersuchte Enzym konnte bezüglich seines Molekulargewichts nicht genau charakterisiert werden. Während Hoskin von einem Molekulargewicht von 26 600 Da ausgegangen ist, halten es andere Forschergruppen z. B. Kopec-Smyth et al. (1993) für wahrscheinlich, daß es sich bei dem 26,6 kDa-Protein nur um ein Fragment eines sehr instabilen 42 kDa Protein handelt. Eine kurze Aminosäuresequenz eines Peptidfragments dieser DFPase wurde 1993 von Ward und Deschamps publiziert. Der dort beschriebene Sequenzabschnitt findet sich nicht in der beanspruchten Aminosäuresequenz der vorliegenden Erfindung wieder, weshalb davon auszugehen ist, daß sich die DFPase aus *Loligo pealii* deutlich von der DFPase der vorliegenden Erfindung unterscheidet.

Außer in *Loligo pealii* wurden ähnliche Proteine in unterschiedlichsten Organismen identifiziert. So fand man sie u. a. in thermophilen oder halophilen Bakterien, in *E. coli, Proteus vulgaris, Saccharomyces cerevisiae, Pseudomonas diminuta, Flavobacterium* und dem eukaryontischen Einzeller *Tetrahymena thermophila*, sowie in Insekten und Invertebraten. Ebenso gelang der Nachweis in Säugetieren wie Mäusen, Ratten, Kaninchen, Schweinen und dem Menschen. Trotz der Anstrengung einer Vielzahl von Arbeitsgruppen, die sich mit Enzymen beschäftigen, die in ihrer Spezifität der DFPase aus *Loligo vulgaris* der vorliegenden Erfindung ähneln, gelang es keiner dieser Gruppen, ein der vorliegenden Erfindung entsprechendes Enzym hinsichtlich seiner Aminosäuresequenz und der dieser zugrundeliegenden Basensequenz vollständig zu klären, die entsprechende Basensequenz mittels eines Vektors in eine Wirtszelle zu transformieren und somit die großtechnische Produktion in hervorragender Reinheit zu ermöglichen. Dierl (1995, Dissertation Johann Wolfgang Goethe Universität, Frankfurt/Main) gelang zwar im Rahmen seiner Forschungsarbeit die Aufklärung einer Teilsequenz der DFPase aus *Loligo vulgaris,* Angaben zur Aminosäuresequenz oder Basensequenz sind dieser Arbeit jedoch nicht zu entnehmen. Die von Dierl zur Verfügung gestellte Teilinformation ist unvollständig und eine Nacharbeitbarkeit der dort vorgestellten Ergebnisse ist bereits deshalb nicht möglich, weil der Öffentlichkeit die der Arbeit zugrundeliegende, jedoch nicht öffentlich zugängliche cDNA-Genbank, nicht zur Verfügung steht.

Eine der Aufgaben der vorliegenden Erfindung besteht darin, das komplette und funktionstüchtige DFP-spaltende Enzym aus *Loligo vulgaris* sowie die das Enzym kodierende Basensequenz zur Verfügung zu stellen, um eine großtechnische Produktion gentechnischer Art zu ermöglichen. Eine weitere Aufgabe war es, eine DFPase bereitzustellen, welche ohne Stabilitätsverlust durch fraktionierte Ammoniumsulfatfüllung in technischem Maßstab isolierbar ist und ihr Aktivitätsoptimum bei einem neutralen pH-Wert von etwa 7,5 und Raumtemperatur (etwa 25 °C) besitzt. Hierdurch sollte nicht zuletzt die Aufgabe einer umweltfreundlichen, energiesparenden Entsorgung von Nervengasen und Insektiziden erreicht werden. Des weiteren war die Aufgabe zu lösen, eine lager- und lösemittelstabile DFPase bereitzustellen. So sollte z. B. eine konzentrierte Lösung über längere Zeit bei 4 °C ohne nennenswerten Aktivitätsverlust stabil sein. Auch sollten verschiedene Lösemittel oder lösemittelhaltige wäßrige Medien (z. B. 10 %ige wäßrige ethanolische Lösung) keinen Einfluß auf die Aktivität des Enzyms besitzen. Es war ferner die Aufgabe zu lösen, eine DFPase bereitzustellen, die in verschiedensten Puffersystemen aktiv ist. Die zur Verfügungstellung der DFPase sollte des weiteren eine Dekontaminierung, Detoxifizierung aber auch Detektion von Acetylcholinesterase-Hemmstoffen, die der DFPase als Substrat dienen, ermöglichen. Die Zurverfügungstellung eines derartigen Enzyms soll nicht nur die großtechnische Vernichtung von entsprechenden Nervengasen oder Insektiziden ermöglichen, sondern auch die Möglichkeit eröffnen, verseuchte Lebensräume (Böden, Gewässer etc.) zu dekontaminieren. Auch eine Klonierung in Pflanzen ist hierdurch möglich. Ferner ist denkbar, das Enzym zur Herstellung von Arzneimitteln zur Entgiftung oder Behandlung von Mensch und Tier einzusetzen. Hierbei könnte das Enzym z. B. lokal in Form einer Hautcreme, parenteral in Form einer Infusions- odex Inhalationslösung oder aber peroral eingesetzt werden.

Die genannten Aufgaben wurden durch Bereitstellung einer DFPase der folgenden Aminosäuresequenz gelöst.


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310

Die beanspruchte Diisopropylfluorophosphatase umfaßt hierbei auch Aminosäure-sequenzen, die durch eine Deletion, Insertion und/oder Substitution einer oder mehrerer Aminosäuren aus obiger Sequenz ableitbar sind, sofern die DFPase Aktivität erhalten bleibt. Dies schließt selbstverständlich auch eine Verkürzung der aminound/oder carboxyterminalen Seite ein.

Dieser erfindungsgemäßen Aminosäuresequenz liegt eine erfindungsgemäße DNA-Sequenz zugrunde, die eine DNA-Sequenz umfaßt, welche für die DFPase aus *Loligo vulgaris* kodiert und welche in einer bevorzugten Ausführungsform folgende Basensequenz umfaßt:

ATGGAAATTC CAGTTATCGA ACCTCTTTTC ACAAAAGTGA CCGAAGATAT ACCAGGTGCA 60
GAGGGTCCCG TTTTTGACAA AAATGGCGAT TTTTATATCG TGGCCCCCGA AGTTGAAGTT 120
AACGGAAAAC CGGCGGGAGA AATTCTACGA ATCGATTTGA AAACAGGAAA GAAAACTGTG 180
ATCTGCAAAC CAGAAGTTAA TGGTTATGGA GGAATTCCTG CTGGCTGCCA ATGTGATCGA 240
GATGCCAACC AGCTGTTTGT GGCCGACATG AGACTCGGCT TGTTGGTCGT GCAAACTGAT 300
GGGACCTTTG AAGAGATTGC CAAAAAAGAC TCTGAAGGTA GAAGAATGCA GGGATGCAAT 360
GATTGCGCAT TTGATTATGA AGGTAACTTG TGGATCACTG CACCAGCTGG GGAAGTCGCA 420
CCTGCAGACT ACACCCGTTC AATGCAGGAA AAATTTGGCA GTATTTACTG CTTCACAACA 480
GATGGTCAAA TGATTCAAGT GGATACTGCT TTCCAGTTTC CAAATGGTAT TGCTGTTGGT 540
CACATGAACG ATGGCCGTCC TTACCAACTA ATTGTGGCTG AAACTCCAAC CAAGAAACTC 600
TGGAGTTATG ATATCAAAGG TCCAGCAAAG ATTGAAAACA AGAAAGTGTG GGGTCACATC 660
CCAGGTACTC ATGAAGGTGG TGCTGATGGA ATGGATTTTG ATGAAGACAA TAACCTTTTG 720
GTAGCCAACT GGGGGAGCTC ACACATCGAA GTGTTCGGCC CAGATGGGGG ACAGCCTAAA 780
ATGAGAATCC GTTGCCCATT TGAAAAACCC AGCAACTTGC ATTTCAAGCC CCAGACCAAA 840
ACCATTTTTG TCACGGAACA CGAGAACAAT GCTGTCTGGA AGTTTGAATG GCAAAGAAAT 900
GGCAAAAAAC AGTATTGTGA GACGTTAAAA TTTGGAATAT TT 942

Durch Veränderungen der Basensequenz können z. B. die Substratspezifität, Löslichkeit und/oder Stabilität des Enzyms den auftretenden Problemstellungen angepaßt werden.

Eine weitere Aufgabe der vorliegenden Erfindung besteht darin, einen Vektor zur Verfügung zu stellen, der mindestens eine Kopie einer erfindungsgemäßen DNA-Sequenz enthält. Dieser Vektor kann ein beliebiger prokaryontischer oder eukaryontischer Vektor sein, auf dem sich die erfindungsgemäße DNA-Sequenz vorzugsweise unter Kontrolle eines Expressionssignals befindet. Zu den Expressionssignalen gehören u. a. Promotoren, Operatoren und Enhancer. Promotoren können z. B. ein T7-Promotor oder lac-, tac-, lacUV5- oder P_{L}-Promotoren sein. Geeignete prokaryontische Vektoren sind z. B. chromosomale Vektoren, wie etwa Bacteriophagen (z. B. Bacteriophage λ) und extrachromosomale Vektoren, wie etwa Plasmide, wobei hierbei zirkuläre Plasmidvektoren bevorzugt sind. Der erfindungsgemäße Vektor kann andererseits auch ein eukaryontischer Vektor sein, z. B. ein Hefevektor oder ein für höhere Zellen geeigneter Vektor, wie etwa ein Plasmidvektor oder ein viraler Vektor. Vektoren der bezeichneten Arten sind dem Fachmann auf dem Gebiet der Molekularbiologie geläufig, so daß an dieser Stelle nicht näher darauf eingegangen werden muß. In einer bevorzugten Ausführungsform können als derartige Vektoren sogenannte Sekretionsvektoren dienen, die die Expression eines Fusionsproteins zwischen DFPase und einem Signalpeptid ermöglichen, wobei das Signalpeptid dafür verantwortlich ist, daß das Protein durch die Zellmembran geschleust wird und bei der Sekretion durch Signalpeptidasen exakt von der DFPase getrennt wird. Als Signalpeptide kommen z. B. ein pelB-, α- oder PH01-Signalpeptid in Frage. Die Verknüpfung der DFpase mit derartigen Signalpeptiden ist vorteilhaft, um die Bildung von "Inclusion bodies", d. h. DFPase- Aggregaten innerhalb der Zelle zu verhindern und gräßere Mengen an Protein in den periplasmatischen Raum oder das Medium zu schleusen.

Die vorliegende Erfindung betrifft des weiteren eine Zelle, die mit einer erfindungsgemäßen DNA-Sequenz oder einem erfindungsgemäßen Vektor transformiert ist. Diese Zelle kann z.B. eine prokaryontische Zelle sein, vorzugsweise eine gramnegative prokaryontische Zelle, besonders bevorzugt eine Eubakterienzelle sein. in einer Ausführungsform ist diese Zelle eine *E. coli*-Zelle. Die Transformation prokaryontischer Zellen mit exogenen Nucleinsäuresequenzen ist dem Fachmann auf dem Gebiet der Molekularbiologie geläufig. Die erfindungsgemäße Zelle kann aber auch eine eukaryontische Zelle sein wie etwa eine Pilzzelle (z. B. Hefe), eine tierische oder pflanzliche Zelle. Bevorzugte eukaryontische Expressionssysteme sind z. B. *Pichia pastoris* oder *Saccharomyces cerevisiae.* Die Transformation bzw. Transfektion eukaryontischer Zellen mit exogenen Nucleinsäuresequenzen ist dem Fachmann auf dem Gebiet der Molekularbiologie geläufig und muß dahex nicht näher erläutert werden.

Durch die Bereitstellung der o. g. Proteine, DNA-Sequenzen, Vektoren und transformierten Zellen wird die großtechnische Produktion eines Enzyms mit DFPase-Aktivität möglich. Hierdurch wird erstmals die Möglichkeit eröffnet, auf einfache, wirtschaftliche und umweltverträgliche Art und Weise große Mengen an Acetylcholinesterase-Hemmstoffen abzubauen.

Hierbei kann die DFPase durch rekombinante DNA-Technologie als Bestandteil eines Extrakts aus dem Wirtsorganismus oder in isolierter und gereinigter Form, z. B. durch Expression in *E. coli* erhalten werden. Die erfindungsgemäße Verwendung einer solchen DFPase besteht im Abbau von P-F-Bindungen oder der Grundformel entsprechenden P-Y-Bindungen enthaltenden Acetylcholinesterase-Hemmstoffen. Vorzugsweise kann hierzu die gereinigte und isolierte DFPase, z. B. zur technischen Spaltung von P-Y-Bindungen (entsprechend der Grundformel) enthaltenden Acetylcholinesterase-Hemmstoffen in einem Enzymreaktor eingesetzt werden. Die Immobilisierung von Enzymen in einem derartigen Reaktor ist dem Fachmann geläufig und muß daher an dieser Stelle nicht ausführlich beschrieben werden. Denkbar ist unter anderem die Einpolymerisierung der DFPase in Schaumstoffe, wie z. B. Polyurethan-Schäume.
Eine andere Ausführungsform der Verwendung ist z. B. der Einsatz der DFPase in einem intakten Mikroorganismus, welcher z. B. zur Dekontamination von Böden im Freiland eingesetzt werden kann, bevorzugt werden hierbei transformierte Bodenbakterien eingesetzt.
Eine weitere erfindungsgemäße Verwendung besteht darin, daß eine erfindungsgemäße DFPase in einem Schaum eingesetzt wird, wobei der Schaum als Träger und/oder Benetzersubstanz wirkt. Ein solcher Schaum kann ein Tensidschaum sein, der u. a. zur Dekontamination von Böden, Flächen, wertvollen Geräten oder dergl. eingesetzt werden kann. In manchen Fällen kann auch ein einfaches Besprühen, also die Applikation als Aerosol ausreichen. Der Einsatz in Schaumform ist dann nicht zwingend notwendig.
Denkbar ist also die Verwendung der DFPase in stationär arbeitenden Verfahren, die sich z.B. Reaktoren bedienen, als auch die Verwendung der DFPase in mobilem Einsatz bei der Dekontamination von Geräten oder großen Freilandflächen. Weitere erfindungsgemäße Ausführungsformen der Verwendung der DFPase können daher auch in der Detoxifizierung belasteter Gewässer oder des Trinkwassers liegen. Eine Immobilisierung des Enzyms zur Verwendung desselben als stationäre Phase in einem Reaktor kann z. B. durch kovalente Verknüpfung des Enzyms an einen festen Träger erfolgen.

Der kodierenden cDNA kann ein "His-Tag" angefügt werden, welches die Produktion einer modifizierten DFPase ermöglicht. Diese modifizierte DFPase ist in der Lage, an Nickel-NTA-modifiziertes Trägermaterial zu binder. Handelt es sich beim Nickel-NTA-modifizierten Trägermaterial um das Füllmaterial einer Trennsäule, so kann diese Methode zur Aufreinigung der DFPase dienen.

Des weiteren können Textilien mit dem Enzym durch kovalente oder nicht-kovalente Verknüpfung imprägniert werden, um als Schutzkleidung zu dienen.

Eine erfindungsgemäße Verwendung kann auch in neuartigen Detektionsmethoden bestehen, z. B. der Biosensorik. Hierbei dient die DFPase als Rezeptor, der auf einem "Transducer" immobilisiert ist. Spaltet die DFPase den zu detektierenden Analyt, d. h. die oben beschriebenen Substrate, so wird das biologische Signal in ein entsprechendes meßbares elektrisches Signal umgewandelt, das durch eine elektronische Komponente verstärkt wird. Das Endsignal steht im allgemeinen im Zusammenhang mit der Menge und/oder Art des Analyten. Die DFPase als Rezeptor kann hierbei in reiner isolierter Form oder in der sie exprimierenden Zelle vorliegen. Als "Transducer" kommen die dem Fachmann auf dem Gebiet der Biosensorik bekannten Überträger-Komponenten in Frage.

Eine weitere erfindungsgemäße Verwendung liegt in der Bereitstellung von Arzneimitteln, welche als Wirkstoff die DFPase enthalten und dadurch in der Lage sind, zu einer Entgiftung bzw. Behandlung eines P-Y-Bindungen enthaltenden Acetylcholinesterase-Hemmstoff vergifteten Menschen oder Tiers beizutragen. Hierbei kommt neben der lokalen Applikation auch die parenterale oder perorale Applikation in Frage.

Ein weiterer Gegenstand der vorliegenden Erfindung ist ein Verfahren zur Herstellung einer DFPase, bei welchem diese durch eine erfindungsgemäße Zelle produziert wind. Dieses Verfahren steht damit einer chemischen Komplett-Synthese der DFPase gegenüber. Beim erfindungsgemäßen Verfahren der Herstellung einer DFPase durch eine transformierte Zelle gehen der Transformation mit einem Expressionsvektor folgende Arbeitsschritte voraus,
(1) Isolierung der mRNA von *Loligo vulgaris* aus deren sofort nach der Tötung entnommenen Kopfganglien, welche sofort in flüssigem Stickstoff tiefzufrieren sind,
(2) Aufreinigung der mRNA mittels Affinitätschromatographie an einer oligo-(dT)-Cellulosesäule,
(3) Übersetzung der mRNA in cDNA,
(4) Klonierung der cDNA in λ-Phagen,
(5) Auffinden der die genetische Information der DFPase enthaltenden λ-Phagen mittels degenerierter Sonden, unter Verwendung der Polymerase-Kettenreaktion,
(6) Amplifikation der genetischen Information mittels der Polymerase-Kettenreaktion,
(7) Überführen der genetischen Information in einen Expressionsvektor.

Bei diesem Verfahren sind verschiedene Schwierigkeiten zu überwinden gewesen, die der Fachmann auf dem Gebiet der Gentechnik nicht mit den ihm zur Verfügung stehenden Standardtechniken hätte lösen können. So war es z. B. nicht offensichtlich, jedoch zwingend notwendig, daß die Isolierung der mRNA aus *Loligo vulgari*s aus den Kopfganglien eines frisch getöteten Tieres erfolgt, wobei die Kopfganglien sofort nach ihrer Entnahme in flüssigem Stickstoff zu lagern sind. Verfährt man nicht in dieser Weise, sind die Chancen, eine "full length"-mRNA der DFPase zu isolieren, nahezu null. Die geringe Anzahl der DFPase kodierenden mRNA-Moleküle macht es darüber hinaus zwingend notwendig, die Technik der Polymerase-Kettenreaktion anzuwenden. Zum Auffinden der genetischen Information in der cDNA-Bank mußten des weiteren hochspezifische Sonden entwickelt werden, die in langen Bereichen mit der cDNA aus *Loligo vulgaris* übereinstimmen mußten. Die Synthese dieser Sonden wurde darüber hinaus dadurch erschwert, daß zum Zeitpunkt der Erfindung keine längeren Proteinsequenzen der DFPase aus *Loligo vulgaris* bekannt waren, welche es ermöglicht hätten, hochspezifische Sonden zu synthetisieren. Erst durch die Sequenzinformation, die in der vorliegenden Erfindung zur Verfügung gestellt wind, ist der Fachmann auf dem Gebiet der Molekularbiologie in der Lage, derartige Sonden in einfacher Weise zu synthetisieren.

Das folgende Beispiel soll zur Veranschaulichung der Erfindung dienen.

### Beispiel

Isolierung der Kopfganglien aus Loligo volgaris bis zur Expression rekombinanten Proteins in Escherichia coli:
(1) Zur Isolierung der Kopfganglien wird der Kopf des Tintenfisches vom Eingeweidesack getrennt und die Tentakeln werden entfernt. Anschließend wird die Epidermis leicht angehoben, um einen Schnitt anzusetzen, der von der Mundhöhle zum Hinterkopf führt. Die Kopfkapsel und das Auge werden freigelegt. Nach Entfernung sämtlicher Nervenstränge wird das Gehirngewebe entnommmen. Sofort nach der Isolierung wird das Gehirngewebe in flüssigen Stickstoff überführt und bis zur RNA-Präparation bei -196 °C gelagert.
   Alle Glasgeräte werden für 4 Stunden bei 250 °C getrocknet. Alle Lösungen werden soweit wie möglich mit Diethylpyrocarbonat behandelt. Das Gehirngewebe wird in einen mit flüssigem Stickstoff gefüllten Mörser überführt und zu einer homogenen Paste zerrieben. Das Gewebe wird anschließend in einen Citratpuffer überführt, der Guanidiumthiocyanat, β-Mercaptoethanol und N-Lauroylsarcosin enthält. Zur Auflösung kleiner noch vorhandener Zellstücke wird das gewonnene Zellysat in einem Glaspotter homogenisiert. Vor Durchführung der Ultrazentrifugation werden noch vorhandene Gewebeteilchen durch eine Zentrifugation aus dem Homogenisat entfernt. Anschließend wird zur Abtrennung der RNA von den anderen Zellbestandteilen eine Ultrazentrifugation mit einem Cäsiumchloridgradienten durchgeführt. Das Pellet der Zentrifugation wird mit Ethanol gewaschen und nach dem Lösen in Wasser mehrmals mit Phenol/Chloroform extrahiert. Zur Kontrolle von Qualität und Quantität der RNA werden übliche Nachweise durchgeführt.
(2) Die Anreicherung der mRNA erfolgt durch eine Affinitätschromatographie an Oligo-(dT)-Cellulose. Hierfür wird nach der Herstellung einer Affinitätschromatographiesäule die Gesamt-RNA erwärmt und anschließend im Eisbad abgekühlt. Nach Auflösen der Sekundärstrukturen wird die Gesamt-RNA bei hohen Salzkonzentrationen auf die Oligo-(dT)-Cellulosesäule aufgetragen. Hierbei wird die Poly-(A⁺)-RNA spezifisch am Trägermaterial gebunden. Um die Ausbeute an mRNA zu erhöhen, wird vorteilhafterweise das Eluat aufgefangen, erneut denaturiert und wiederum aufgebracht. Die Säule mit der daran gebundenen Poly-(A⁺)-RNA wird mit Ladungs- und Waschpuffer gespült. Von der beladenen Oligo-(dT)-Cellulose wird die Poly-(A⁺)-RNA durch eine Lösung mit geringer Salzkonzentration eluiert und die geeigneten Fraktionen spektralphotometrisch bestimmt.
(3) Zur Erststrangsynthese der cDNA wird ein Oligo-(dT)-Primer verwendet, der neben der für die Bindung benötigten Poly-(dT)-Sequenz eine *XhoI-*Schnittstelle und eine "GAGA"-Sequenz enthält. Nach Bildung des Hybrids wird durch die reverse Transkriptase ein zur mRNA komplementärer DNA-Strang synthetisiert. Für diese Reaktion wird wegen ihrer geringeren RNase H-Aktivität, der höheren Prozessivität und der geringeren Inhibierbarkeit durch Poly-(A⁻)-RNA die reverse Transkriptase aus Moloney Mäuseleukämievirus (M-MuL VRT) verwendet. Zum Schutz vor einem Restriktionsenzymverdau in der Synthese wird für den Erststrang ein Gemisch aus dATP, dGTP, dTTP und 5-MethyldCTP verwendet. Nach der Synthese wird für die ersten Replikationsrunden der *Escherichia coli* Bakterienstamm PKL-F' benutzt, der die genetischen Marker *mcrA*^{*-*} und *mcrB*^{*-*} trägt.
   Im Anschluß an die Synthese des Erststrangs der cDNA wird der Zweitstrang nach dem Verfahren von Gubler und Hoffman synthetisiert. Zunächst wird mit der Endoribonuklease RNase H die RNA des RNA:DNA-Doppelstrangs gespalten. Hierbei entstehen Oligoribonukleotide mit 5'-Phosphat und 3'-Hydroxy-Enden, die von der DNA-Polymerase I erkannt und als Startex der DNA-Synthese benutzt werden.
   Um den erhaltenen DNA-Doppelstrang in einen geeigneten Vektor einklonieren zu können, werden in einem ersten Schritt überhängende Enden der DNA-Stränge durch Behandlung mit T4 DNA-Polymerase aufgefüllt. Anschließend werden mit Hilfe der T4 DNA-Ligase die Enden der cDNA mit *EcoR I*-Adaptoren versehen. Hierbei wird ein Gemisch eines phosphorylierten 9merund eines dephosphorylierten 13mer-Oligodesoxyribonukleotids verwendet. Nach Hitzeinaktivierung der Ligase werden die überstehenden 5'-Enden durch die T4 Polynukleotid-Kinase phosphoryliert und der DNA-Doppelstrang mit dem Restriktionsenzym *XhoI* nachgeschnitten. Durch die Verwendung zweier unterschiedlicher Restriktionsschnittstellen an den Enden der cDNA ist eine gerichtete Klonierung in einen geeigneten Vektor möglich.
(4) Die erhaltene cDNA wird in die Vektorarme des λ-Bakteriophagen einkloniert. Durch *in vitro*-Verpackung in vorgefertigte Phagenköpfe und Inkubation mit unterschiedlichen Zellextrakten von Phagenmutanten erhält man lebensfähige Bakteriophagen. Es werden zwei cDNA-Banken mit unterschiedlich hoher Anzahl von rekombinaten Vektormolekülen erstellt, die als Ausgangspunkte der weiteren Arbeiten dienen.
(5) Zur Durchmusterung der beiden cDNA-Banken nach der genetischen Information der Diisopropylfluorophosphatase werden aus der zuvor aufgeklärten Teilinformation der Aminosäuresequenz des Proteins - unter Zuhilfenahme weiterer Techniken - Oligodesoxyribonukleotide entworfen, die eine größtmögliche Übereinstimmung mit der cDNA-Sequenz des Proteins haben sollten. Unter Verwendung der Polymerase-Kettenreaktion können mit einem sequenzspezifischen Oligodesoxyribonukleotid - der Sequenz 5'-TTC-CAA-TTC-CCI-AAT-GGI-ATT-GCT-GT-3' - unter sehr stringenten Bedingungen Produkte erzeugt werden. In den PCR-Experimenten zur Identifikation der Diisopropylfluorophosphatase aus *Loligo vulgaris* werden zwei Oligodesoxyribonukleotide verwendet. Das erste Oligodesoxyribonukleotid besteht aus einer Sequenz des λ-Bakteriophagen, welche an die einklonierte cDNA-Sequenz anschließt. Das zweite Oligodesoxyribonukleotid, welches in einigen Positionen Inosin enthält, leitet sich aus der aufgeklärten Proteinsequenz ab und ist deshalb für die Diisopropylfluorophosphatase aus *Loligo vulgaris* spezifisch.
In den cDNA-Banken kann ein Produkt mit einer Länge von 550 Bp aufgespürt werden. Nach Isolierung und Aufreinigung des Produkts der Polymerase-Kettenreaktion kann die enthaltene DNA-Information in Sequenzierversuchen entschlüsselt werden. Durch die Verwendung von Oligodesoxyribonukleotiden, die aus der bereits bekannten DNA-Sequenz abgeleitet sind, kann die Sequenzierung beider DNA-Stränge des cDNA-Inserts erfolgen. Durch Vergleich des aus der DNA-Sequenzierung ermittelten Leserasters mit den zuvor bestimmten Proteinsequenzen kann nachgewiesen werden, daß es sich bei dem untersuchten 550 Bp-Fragment um eine Teilsequenz der gesuchten Diisopropylfluorophosphatase aus *Loligo vulgaris* handelt.
   Ausgehend von dieser Teilsequenz des Enzyms kann die gesamte cDNA-Sequenz der Diisopropylfluorophosphatase mit einer Gesamtlänge von 1210 Bp aufgeklärt werden. Charakterisiert wird diese DNA-Sequenz durch einen etwa 210 Bp langen Bereich am 5'-Ende, der keine kodierende DNA-Sequenz enthält. Der offene Leseraster besteht aus 942 Bp und kodiert für ein Protein mit 314 Aminosäuren, das ein Molekulargewicht von ungefähr 35 kDa besitzt.
(6) Zum Aufbau eines Expressionssystems wird der offene Leserahmen des Enzyms mittels flankierender Oligodesoxyribonukleotide in einer Polymerase-Kettenreaktion amplifiziert. Zusätzlich zu der sequenzspezifischen Information des Proteins enthalten die Oligodesoxyribonukleotide Restriktionsschnittstellen, die eine Klonierung in ein Expressionssystem vereinfachen. Auf der 5'-Seite der Information wird eine *Nco I*-Schnittstelle, auf der 3'-Seite der DNA eine *Hind III*-Schnittstelle eingefügt. Das Produkt der Polymerase-Kettenreaktion wird mittels der *Pfu*-Taq-Polymerase "gebluntet", über ein Agarosegel gereinigt, isoliert und in den geöffneten Vektor pCR-Script SK(+) umgesetzt.
(7) Nach Selektion der Plasmide, die die gesuchte genetische Information enthalten, wird diese mit *Nco I*/*Hind III* herausgeschnitten und in einen Expressionsvektor mit *trc*-Promotorsystem umgesetzt. Durch Expression in diesem System können 150 - 200 mg rekombinante und biologisch aktive Diisopropylfluorophosphatase pro Liter Medium gewonnen werden.

## Patentansprüche

1. Isolierte Diisopropylfluorophosphatase, **gekennzeichnet durch** nachstehende Aminosäuresequenz 

1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310
 oder eine **durch** Deletion, Insertion und/oder Substitution einer oder mehrerer Aminosäuren ableitbare Sequenz.

2. Diisopropylfluorophosphatase, **gekennzeichnet durch** nachstehende Aminosäuresequenz


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310

3. DNA-Sequenz, die eine DNA-Sequenz umfaßt, die für ein Polypeptid mit einer DFPase-Aktivität kodiert, wobei das kodierte Polypeptid nachstehende Aminosäure-Sequenz 

1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310
 oder eine durch Deletion, Insertion und/oder Substitution einer oder mehrerer Aminosäuren ableitbare Sequenz besitzt.

4. DNA-Sequenz gemäß Anspruch 3, die folgende Basensequenz enthält oder umfaßt:

ATGGAAATTC CAGTTATCGA ACCTCTTTTC ACAAAAGTGA CCGAAGATAT ACCAGGTGCA 60
GAGGGTCCCG TTTTTGACAA AAATGGCGAT TTTTATATCG TGGCCCCCGA AGTTGAAGTT 120
AACGGAAAAC CGGCGGGAGA AATTCTACGA ATCGATTTGA AAACAGGAAA GAAAACTGTG 180
ATCTGCAAAC CAGAAGTTAA TGGTTATGGA GGAATTCCTG CTGGCTGCCA ATGTGATCGA 240
GATGCCAACC AGCTGTTTGT GGCCGACATG AGACTCGGCT TGTTGGTCGT GCAAACTGAT 300
GGGACCTTTG AAGAGATTGC CAAAAAAGAC TCTGAAGGTA GAAGAATGCA GGGATGCAAT 360
GATTGCGCAT TTGATTATGA AGGTAACTTG TGGATCACTG CACCAGCTGG GGAAGTCGCA 420
CCTGCAGACT ACACCCGTTC AATGCAGGAA AAATTTGGCA GTATTTACTG CTTCACAACA 480
GATGGTCAAA TGATTCAAGT GGATACTGCT TTCCAGTTTC CAAATGGTAT TGCTGTTCGT 540
CACATGAACG ATGGCCGTCC TTACCAACTA ATTGTGGCTG AAACTCCAAC CAAGAAACTC 600
TGGAGTTATG ATATCAAAGG TCCAGCAAAG ATTGAAAACA AGAAAGTGTG GGGTCACATC 660
CCAGGTACTC ATGAAGGTGG TGCTGATGGA ATGGATTTTG ATGAAGACAA TAACCTTTTG 720
GTAGCCAACT GGGGGAGCTC ACACATCGAA GTGTTCGGCC CAGATGGGGG ACAGCCTAAA 780
ATGAGAATCC GTTGCCCATT TGAAAAACCC AGCAACTTGC ATTTCAAGCC CCAGACCAAA 840
ACCATTTTTG TCACGGAACA CGAGAACAAT GCTGTCTGGA AGTTTGAATG GCAAAGAAAT 900
GGCAAAAAAC AGTATTGTGA GACGTTAAAA TTTGGAATAT TT 942

5. Vektor, **dadurch gekennzeichnet, daß** er mindestens eine Kopie einer DNA-Sequenz nach einem der Ansprüche 3 oder 4 enthält.

6. Zelle, **dadurch gekennzeichnet, daß** sie mit einer DNA-Sequenz nach Anspruch 3 oder 4 oder einem Vektor nach Anspruch 5 transformiert ist.

7. Zelle nach Anspruch 6, die eine transformierte E. coli-Zelle ist.

8. Zelle nach Anspruch 6, die ein Bodenbakterium oder ein eukaryontisches Zellsystem ist.

9. Verfahren zur Herstellung einer DFPase gemäß Anspruch 1 oder 2, bei welchem diese durch eine Zelle gemäß einem der Ansprüche 6, 7 oder 8 produziert wird

10. Verwendung einer DFPase nach Anspruch 1 oder 2 zum Abbau von P-Y-Bindungen enthaltenden Acetylcholinesterasehemmstoffen der folgenden Grundstruktur 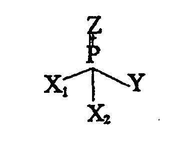 wobei Z für Sauerstoff oder Schwefel, Y für eine Säureanhydridgruppe (deprotonierte Säuregruppe), insbesondere eine F- oder CN-Gruppe oder für eine Estergruppe, insbesondere eine Thioester-, Enolester- oder p-Nitrophenylestergruppe, sowie X₁ und X₂ für eine geradkettige, verzweigte oder cyclische, 1 bis 15 Kohlenstoffatome enthaltende, Alkoxy-, Alkyl-, Aryl-, Alkylamino- oder Dialkylaminogruppe steht, und X₁ und X₂ gleich oder verschieden sind.

11. Verwendung gemäß Anspruch 10, wobei die DFPase auf oder in einem Trägermaterial immobilisiert ist.

12. Verwendung gemäß Anspruch 10 oder 11, wobei die DFPase in einem Enzymreaktor eingesetzt wird.

13. Verwendung gemäß Anspruch 11, wobei die DFPase in Substanz oder in einem immobilisierten transformierten DFPase produzierenden Mikroorganismus vorliegt und die DFPase als Rezeptor in einem Biosensoraufbau fungiert.

14. Verwendung gemäß Anspruch 10, wobei die DFPase in Form einer ein Aerosol bildenden Sprühflüssigkeit zur Dekontamination eingesetzt wird.

15. Verwendung gemäß Anspruch 10, wobei die DFPase in einem Tensidschaum zur Dekontamination eingesetzt wird.

16. Verwendung gemäß einem der Ansprüche 14 oder 15, wobei Böden oder Geräte dekontaminiert werden.

17. Verwendung gemäß Anspruch 10, wobei Trinkwasser oder Gewässer dekontaminiert werden.

18. Verwendung gemäß Anspruch 10, zur Herstellung eines Arzneimittels gegen Vergiftung mit P-Y-Bindungen enthaltenden Acetylcholinesterasehemmern der folgenden Grundstruktur 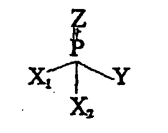 wobei Z für Sauerstoff oder Schwefel, Y für eine Säureanhydridgruppe (deprotonierte Säuregruppe), insbesondere eine F- oder CN-Gruppe oder für eine Estergruppe, insbesondere eine Thioester-, Enolester- oder p-Nitrophenylestergruppe, sowie X₁ und X₂ für eine geradkettige, verzweigte oder cyclische, 1 bis 10 Kohlenstoffatome enthaltende, Alkoxy-, Alkyl-, Aryl-, Alkylamino- oder Dialkylaminogruppe steht, und X₁ und X₂ gleich oder verschieden sind.

19. Verwendung gemäß Anspruch 18, wobei sich das Arzneimittel zur lokalen, parenteralen oder peroralen Applikation eignet.

20. Verwendung gemäß Anspruch 10, wobei das Trägermaterial ein textiles Material ist.

## Claims

1. Isolated Diisopropyl-fluorophosphatase, **characterized by** the following amino acid sequence 

1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310
 or a sequence which can be derived by deletion, insertion and/or substitution of one or more amino acids.

2. Diisopropyl-fluorophosphatase, **characterized by** the following amino acid sequence

Met Glu Ile Pro Val Ile Glu Pro Leu Phe Thr Lys Val Thr Glu Asp
1               5                   10                  15

Ile Pro Gly Ala Glu Gly Prc Val Phe Asp Lys Asn Gly Asp Phe Tyr
            20                  25                  30

Ile Val Ala Pro Glu Val Glu Val Asn Gly Lys Pro Ala Gly Glu Ile
        35                  40                  45

Leu Arg Ile Asp Leu Lys Thr Gly Lys Lys Thr Val Ile Cys Lys Pro
    50                  55                  60

Glu Val Asn Gly Tyr Gly Gly Ile Pro Ala Gly Cys Gln Cys Asp Arg
65                  70                  75                  80

Asp Ala Asn Gln Leu Phe Val Ala Asp Met Arg Leu Gly Leu Leu Val
                85                  90                  95

Val Gln Thr Asp Gly Thr Phe Glu Glu Ile Ala Lys Lys Asp Ser Glu
            100                 105                 110

Gly Arg Arg Met Gln Gly Cys Asn Asp Cys Ala Phe Asp Tyr Glu Gly
        115                 120                 125

Asn Leu Trp Ile Thr Ala Pro Ala Gly Glu Val Ala Pro Ala Asp Tyr
    130                 135                 140

Thr Arg Ser Met Gln Glu Lys Phe Gly Ser Ile Tyr Cys Phe Thr Thr
145                 150                 155                 160

Asp Gly Gln Met Ile Gln Val Asp Thr Ala Phe Gln Phe Pro Asn Gly
                165                 170                 175

Ile Ala Val Arg His Met Asn Asp Gly Arg Pro Tyr Gln Leu Ile Val
            180                 185                 190

Ala Glu Thr Pro Thr Lys Lys Leu Trp Ser Tyr Asp Ile Lys Gly Pro
        195                 200                 205

Ala Lys Ile Glu Asn Lys Lys Val Trp Gly His Ile Pro Gly Thr His
    210                 215                 220

Glu Gly Gly Ala Asp Gly Met Asp Phe Asp Glu Asp Asn Asn Leu Leu
225                 230                 235                 240

Val Ala Asn Trp Gly Ser Ser His Ile Glu Val Phe Gly Pro Asp Gly
                245                 250                 255

Gly Gln Pro Lys Met Arg Ile Arg Cys Pro Phe Glu Lys Pro Ser Asn
            260                 265                 270

Leu His Phe Lys Pro Gln Thr Lys Thr Ile Phe Val Thr Glu His Glu
        275                 280                 285

Asn Asn Ala Val Trp Lys Phe Glu Trp Gln Arg Asn Gly Lys Lys Gln
    290                 295                 300

Tyr Cys Glu Thr Leu Lys Phe Gly Ile Phe
305                 310

3. DNA sequence which comprises a DNA sequence which codes for a polypeptide having a DFPase activity, where the encoded polypeptide has the following amino acid sequence 

1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310
 or a sequence which can be derived by deletion, insertion and/or substitution of one or more amino acids.

4. DNA sequence according to Claim 3, which contains or comprises the following base sequence:

ATGGAAATTC CAGTTATCGA ACCTCTTTTC ACAAAAGTGA CCGAAGATAT ACCAGGTGCA 60
GAGGGTCCCG TTTTTGACAA AAATGGCGAT TTTTATATCG TGGCCCCCGA AGTTGAAGTT 120
AACGGAAAAC CGGCGGGAGA AATTCTACGA ATCGATTTGA AAACAGGAAA GAAAACTGTG 180
ATCTGCAAAC CAGAAGTTAA TGGTTATGGA GGAATTCCTG CTGGCTGCCA ATGTGATCGA 240
GATGCCAACC AGCTGTTTGT GGCCGACATG AGACTCGGCT TGTTGGTCGT GCAAACTGAT 300
GGGACCTTTG AAGAGATTGC CAAAAAAGAC TCTGAAGGTA GAAGAATGCA GGGATGCAAT 360
GATTGCGCAT TTGATTATGA AGGTAACTTG TGGATCACTG CACCAGCTGG GGAAGTCGCA 420
CCTGCAGACT ACACCCGTTC AATGCAGGAA AAATTTGGCA GTATTTACTG CTTCACAACA 480
GATGGTCAAA TGATTCAAGT GGATACTGCT TTCCAGTTTC CAAATGGTAT TGCTGTTCGT 540
CACATGAACG ATGGCCGTCC TTACCAACTA ATTGTGGCTG AAACTCCAAC CAAGAAACTC 600
TGGAGTTATG ATATCAAAGG TCCAGCAAAG ATTGAAAACA AGAAAGTGTG GGGTCACATC 660
CCAGGTACTC ATGAAGGTGG TGCTGATGGA ATGGATTTTG ATGAAGACAA TAACCTTTTG 720
GTAGCCAACT GGGGGAGCTC ACACATCGAA GTGTTCGGCC CAGATGGGGG ACAGCCTAAA 780
ATGAGAATCC GTTGCCCATT TGAAAAACCC AGCAACTTGC ATTTCAAGCC CCAGACCAAA 840
ACCATTTTTG TCACGGAACA CGAGAACAAT GCTGTCTGGA AGTTTGAATG GCAAAGAAAT 900
GGCAAAAAAC AGTATTGTGA GACGTTAAAA TTTGGAATAT TT 942

5. Vector, **characterized in that** it contains at least one copy of a DNA sequence according to either of Claims 3 or 4.

6. Cell, **characterized in that** it is transformed with a DNA sequence according to Claim 3 or 4 or a vector according to Claim 5.

7. Cell according to Claim 6, which is a transformed E. *coli* cell.

8. Cell according to Claim 6, which is a soil bacterium or a eukaryotic cell system.

9. Method for the production of a DFPase according to Claim 1 or 2, in which the latter is produced by a cell according to any of Claims 6, 7 or 8.

10. Use of a DFPase according to Claim 1 or 2 for degradation of acetylcholinesterase inhibitors containing P-Y linkages and having the following basic structure 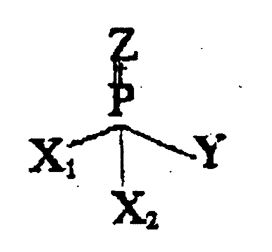 where Z represents oxygen or sulphur, Y represents an anhydride group (deprotonated acid group), in particular an F or CN group or represents an ester group, in particular a thioester, enol ester or p-nitrophenyl ester group, and X₁ and X₂ represent a straight-chain, branched or cyclic alkoxy, alkyl, aryl, alkylamino or dialkylamino group containing 1 to 15 carbon atoms, and X₁ and X₂ are identical or different.

11. Use according to Claim 10, where the DFPase is immobilized on or in a carrier material.

12. Use according to Claim 10 or 11, where the DFPase is employed in an enzyme reactor.

13. Use according to Claim 11, where the DFPase is present as such or in an immobilized transformed DFPase-producing microorganism, and the DFPase acts as receptor in a biosensor assembly.

14. Use according to Claim 10, where the DFPase is employed in the form of an aerosol-forming spray liquid for the decontamination.

15. Use according to Claim 10, where the DFPase is employed in a surfactant foam for the decontamination.

16. Use according to either of Claims 14 or 15, where soils or equipment are decontaminated.

17. Use according to Claim 10, where drinking water or watercourses are decontaminated.

18. Use according to Claim 10 for producing a medicinal product to counter poisoning by acetylcholinesterase inhibitors containing P-Y linkages and having the following basic structure 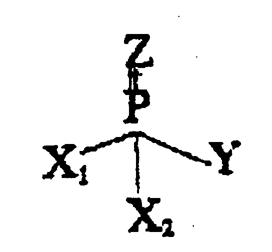 where Z represents oxygen or sulphur, Y represents an anhydride group (deprotonated acid group), in particular an F or CN group or represents an ester group, in particular a thioester, enol ester or p-nitrophenyl ester group, and X₁ and X₂ represent a straight-chain, branched or cyclic alkoxy, alkyl, aryl, alkylamino or dialkylamino group containing 1 to 15 carbon atoms, and X₁ and X₂ are identical or different.

19. Use according to Claim 18, where the medicinal product is suitable for local, parenteral or oral administration.

20. Use according to Claim 10, where the carrier material is a textile material.

## Revendications

1. Diisopropylfluorophosphatase isolée, caractërisée par la séquence d'acides aminés suivante : 

1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310
 ou par une séquence pouvant être obtenue par délétion, insertion et/ou substitution d'un ou plusieurs acides aminés.

2. Diisopropylfluorophosphatase **caractérisée par** la séquence d'acides aminés suivante :


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310

3. Séquence d'ADN qui comprend une séquence d'ADN codant pour un polypeptide ayant une activité DFPase, le polypeptide codé ayant la séquence d'acides aminés suivante : 

1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310
 ou une séquence pouvant être obtenue par délétion, insertion et/ou substitution d'un ou plusieurs acides aminés.

4. Séquence d'ADN selon la revendication 3, **caractérisé en ce qu'**elle contient ou comprend la séquence de bases suivante :

ATGGAAATTC CAGTTATCGA ACCTCTTTTC ACAAAAGTGA CCGAAGATAT ACCAGGTGCA 60
GAGGGTCCCG TTTTTGACAA AAATGGCGAT TTTTATATCG TGGCCCCCGA AGTTGAAGTT 120
AACGGAAAAC CGGCGGGAGA AATTCTACGA ATCGATTTGA AAACAGGAAA GAAAACTGTG 180
ATCTGCAAAC CAGAAGTTAA TGGTTATGGA GGAATTCCTG CTGGCTGCCA ATGTGATCGA 240
GATGCCAACC AGCTGTTTGT GGCCGACATG AGACTCGGCT TGTTGGTCGT GCAAACTGAT 300
GGGACCTTTG AAGAGATTGC CAAAAAAGAC TCTGAAGGTA GAAGAATGCA GGGATGCAAT 360
GATTGCGCAT TTGATTATGA AGGTAACTTG TGGATCACTG CACCAGCTGG GGAAGTCGCA 420
CCTGCAGACT ACACCCGTTC AATGCAGGAA AAATTTGGCA GTATTTACTG CTTCACAACA 480
GATGGTCAAA TGATTCAAGT GGATACTGCT TTCCAGTTTC CAAATGGTAT TGCTGTTCGT 540
CACATGAACG ATGGCCGTCC TTACCAACTA ATTGTGGCTG AAACTCCAAC CAAGAAACTC 600
TGGAGTTATG ATATCAAAGG TCCAGCAAAG ATTGAAAACA AGAAAGTGTG GGGTCACATC 660
CCAGGTACTC ATGAAGGTGG TGCTGATGGA ATGGATTTTG ATGAAGACAA TAACCTTTTG 720
GTAGCCAACT GGGGGAGCTC ACACATCGAA GTGTTCGGCC CAGATGGGGG ACAGCCTAAA 780
ATGAGAATCC GTTGCCCATT TGAAAAACCC AGCAACTTGC ATTTCAAGCC CCAGACCAAA 840
ACCATTTTTG TCACGGAACA CGAGAACAAT GCTGTCTGGA AGTTTGAATG GCAAAGAAAT 900
GGCAAAAAAC AGTATTGTGA GACGTTAAAA TTTGGAATAT TT 942

5. Vector **caractérisé en ce qu'**il comprend au moins une copie de la séquence d'ADN selon l'une des revendications 3 et 4.

6. Cellule **caractérisée en ce qu'**elle est transformée par une séquence d'ADN selon la revendication 3 ou 4 ou par un vector selon la revendication 5.

7. Cellule selon la revendication 6, qui est une cellule de E.coli transformée.

8. Cellule selon la revendication 6, qui est une bactérie du sol ou un système cellulaire eucaryote.

9. Procédé de préparation d'une DFPase selon la revendication 1 ou 2, dans lequel celle-ci est produite par une cellule selon l'une des revendications 6, 7 et 8.

10. Utilisation d'une DFPase selon la revendication 1 ou 2 pour la dégradation d'inhibiteurs de l'acétylcholinestérase contenant des liaisons P-Y-, ayant la structure de base suivante : 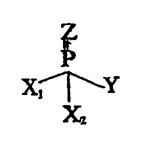 dans laquelle Z représente oxygène ou soufre, Y représente un groupe anhydride d'acide (groupe acide déprotoné), en particulier un groupe F ou CN, ou un groupe ester, en particulier un groupe thioester, énolester ou p-nitrophénylester, et X₁ et X₂ représentent un groupe alkoxy, alkyle, aryle, alkylamino ou dialkylamino ayant de 1 à 15 atomes de carbone, et linéaire, ramifié ou cyclique, X₁ et X₂ étant identiques ou différents.

11. Utilisation selon la revendication 10, dans laquelle la DFPase est immobilisée sur ou dans un support.

12. Utilisation selon la revendication 10 ou 11, dans laquelle la DFPase est mise en oeuvre dans un réacteur enzymatique.

13. Utilisation selon la revendication 11, dans laquelle la DFPase est présente en tant que telle ou dans un microorganisme producteur de DFPase, immobilisé et transformé, la DFPase se comportant comme un récepteur dans la synthèse d'un biocapteur.

14. Utilisation selon la revendication 10, dans laquelle la DFPase est mise en oeuvre sous la forme d'un liquide de pulvérisation formant un aérosol, pour la décontamination.

15. Utilisation selon la revendication 10, dans laquelle la DFPase est mise en oeuvre dans une mousse tensio-active pour la décontamination.

16. Utilisation selon la revendication 14 ou 15, pour la décontamination de sols ou d'appareils.

17. Utilisation selon la revendication 10, pour la décontamination de l'eau potable ou des eaux.

18. Utilisation selon la revendication 10, pour la préparation d'un médicament contre l'intoxication par des inhibiteurs de l'acétylcholinestérase contenant des liaisons P-Y-, de structure de base suivante : 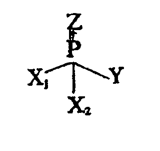 dans laquelle Z représente oxygène ou soufre, Y représente un groupe anhydride d'acide (groupe acide déprotoné), en particulier un groupe F ou CN, ou un groupe ester, en particulier un groupe thioester, énolester ou p-nitrophénylester, et X₁ et X₂ représentent un groupe alkoxy, alkyle, aryle, alkylamino ou dialkylamino ayant de 1 à 10 atomes de carbone, et linéaire, ramifié ou cyclique, X₁ et X₂ étant identiques ou différents.

19. Utilisation selon la revendication 18, dans laquelle le médicament convient pour une application locale, parentérale ou orale.

20. Utilisation selon la revendication 10, dans laquelle le support est un matériau textile.
